(19)

Europäisches
Patentamt
European
Patent Office
Office européen
des brevets

(11) **EP 3 238 619 A1**

(12) **EUROPEAN PATENT APPLICATION**

(43) Date of publication:
**01.11.2017 Bulletin 2017/44**

(51) Int Cl.:
*A61B 5/11* (2006.01)          *G06K 9/00* (2006.01)
*A61B 5/00* (2006.01)

(21) Application number: **16197015.7**

(22) Date of filing: **03.11.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **25.04.2016 TW 105112815**

(71) Applicant: **Cal-Comp Electronics & Communications Company Limited New Taipei City 22201 (TW)**

(72) Inventors:
• **HARAIKAWA, Koichi**
  **22201 New Taipei City (TW)**
• **HUANG, Yu-Shun**
  **22201 New Taipei City (TW)**
• **CHIEN, Jen-Chien**
  **22201 New Taipei City (TW)**
• **GUO, Han-Wen**
  **22201 New Taipei City (TW)**

(74) Representative: **Becker Kurig Straus Patentanwälte Bavariastrasse 7 80336 München (DE)**

(54) **GAIT EVALUATION METHOD AND ELECTRONIC DEVICE THEREOF**

(57)     A gait evaluation method using a sensor worn by the user for evaluating the gait of the user includes the following steps. A plurality of first acceleration values, a plurality of second acceleration values and a plurality of third acceleration values on a first direction, a second direction and a third direction corresponding to the user within a walking period of the user are obtained from the sensor worn by the user. Based on the first acceleration values, the second acceleration values, the third acceleration values and a wearing position of the sensor on the user, a sway index, a step index and a slouch index related to the user are calculated. A gait evaluation is generated according to the sway index, the step index and the slouch index. An electronic device suitable for applying the gait evaluation method is also provided in the present application.

FIG. 1

**Description**

BACKGROUND OF THE INVENTION

Field of the Invention

The present invention relates to an evaluation method and an electronic device, and more particularly relates to a gait evaluation method and an electronic device thereof.

Description of Related Art

In modem life, more and more people are taking care of whether their walking posture (e.g. gait) is correct and good while they are walking, due to the pursuit of health and good deportment. To be specific, not only the perception of others is affected, some symptoms such as back pain, leg pain and joint pain are also caused if someone has a bad walking posture. Accordingly, a variety of devices and methods are proposed to help determine whether a user's walking posture is good.

In tradition, the walking posture of the user can be determined by, for example, image processing technology or human eyes. However, the determination using human eyes may be inaccurate due to the subjective consciousness. Relatively, the determination using image processing technology is time-consuming and bound to occupy a lot of calculation resources. Accordingly, the creation of an easy and accurate gait evaluation method and a related electronic device is still a target for people skilled in the art to work on.

SUMMARY OF THE INVENTION

The present invention provides a gait evaluation method and an electronic device thereof, which calculate a plurality of indexes by detecting changes of acceleration values on each directions corresponding to a user and correct sensing errors, so as to evaluate a gait of the user accurately.

The embodiment of the invention provides a gait evaluation method, which uses a sensor worn by a user to evaluate a gait of the user. The gait evaluation method includes the following steps. Obtaining a plurality of first acceleration values, a plurality of second acceleration values and a plurality of third acceleration values on a first direction, a second direction and a third direction corresponding to the user within a walking period of the user from the sensor worn by the user. Obtaining a wearing position of the sensor on the user. Calculating a sway index related to the user based on the first acceleration values, the second acceleration values and the wearing position of the sensor on the user. Calculating a step index related to the user based on the first acceleration values, the second acceleration values and the third acceleration values. Calculates a slouch index related to the user based on the third acceleration values. Generating a gait evaluation result according to the sway index, the step and the slouch index.

The present invention provides an electronic device adapted to evaluate a gait of a user. The electronic device includes a sensor and a processor coupled to the sensor. The sensor is worn by the user, and detects a plurality of first acceleration values, a plurality of second acceleration values and a plurality of third acceleration values on a first direction, a second direction and a third direction corresponding to the user within a walking period of the user. The processor obtains the first acceleration values, the second acceleration values and the third acceleration values on the first direction, the second direction and the third direction corresponding to the user from the sensor worn by the user, and obtains a wearing position of the sensor on the user. The processor calculates a sway index related to the user based on the first acceleration values, the second acceleration values and the wearing position of the sensor on the user. The processor calculates a step index related to the user based on the first acceleration values, the second acceleration values and the third acceleration values. The processor calculates a slouch index related to the user based on the third acceleration values. The processor generates a gait evaluation result according to the sway index, the step index and the slouch index.

Based on the above, in the gait evaluation method and the related electronic device provided in the embodiments of the invention, a plurality of first acceleration values, a plurality of second acceleration values, a plurality of third acceleration values and a wearing position of a sensor on a user detected within a walking period of the user are used to calculate a plurality of indexes of different types respectively, and a gait evaluation result is generated according to the indexes, so as to evaluate a gait of the user accurately.

To make the above features and advantages of the present invention more comprehensible, several embodiments accompanied with drawings are described in detail as follows.

BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the invention.

FIG. 1 is a schematic diagram of gait features according to an embodiment of the present invention.
Fig. 2 is a block diagram of an electronic device according to an embodiment of the present invention.
FIG. 3 is a flowchart of a gait evaluation method according to an embodiment of the invention.
FIG. 4 is a flowchart of calculating a sway index according to an embodiment of the invention.
FIG. 5 is a schematic diagram of a correction of a sway index according to an embodiment of the present invention.
FIG. 6 is a flowchart of calculating a step index according to an embodiment of the invention.
FIG. 7 is a flowchart of calculating a slouch index according to an embodiment of the invention.

DESCRIPTION OF THE EMBODIMENTS

It is to be understood that both the foregoing and other detailed descriptions, features and advantages are intended to be described more comprehensively by providing an embodiment accompanied with figures hereinafter. In the following embodiments, wordings used to indicate directions, such as "up," "down," "front," "back," "left," and "right", merely refer to directions in the accompanying drawings. Therefore, the directional wording is used to illustrate rather than limit the invention. It should be pointed out first that the same or similar reference numerals or labels represent the same or similar components in the following embodiments.

Some body characteristics are usually used to evaluate whether a gait of an individual is standard. In detail, a standard gait includes the following features. First, a line extended from head, neck to waist is a vertical line. During walking, two tiptoes are pointing forward, and the heel leaves and touches the ground ahead to the sole of the foot. Besides, during walking, two hands are drooping and swinging naturally.

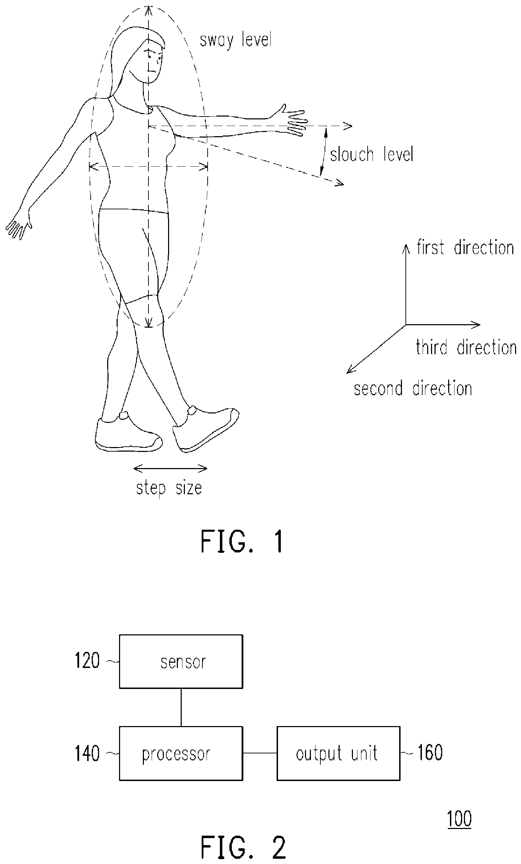

The gait evaluation method and the related electronic device provided in the embodiments of the invention further simplify the gait features into three categories, so as to perform the gait evaluation with reference to the said manner of evaluation. FIG. 1 is a schematic diagram of gait features according to an embodiment of the present invention. Referring to FIG. 1, in the present embodiment, a sway level of a user's body along a first direction and a second direction, a step size of the user along a third direction and a slouch level of the user's body along the third direction are used to evaluate the gait of the user while the user walks along the third direction.

The said gait features are obtained by the way proposed in the embodiments of the invention for detecting changes of accelerations of the user on the first, second and third direction. Fig. 2 is a block diagram of the electronic device according to an embodiment of the present invention. Referring to FIG. 2, the electronic device 100 includes a sensor 120, a processor 140 and an output unit 160.

In the present embodiment, the sensor 120 may include an accelerometer, a gyroscope, an electronic compass or similar devices, which is not limited by the invention. In other embodiments, the sensor 120 may further include other types of sensing devices for sensing physiological signals. To be specific, the sensor 120 may be used for detecting accelerations along three axes, but the functions are not limited thereto.

In the present embodiment, the processor 140 may include a micro-controller, an embedded controller, a central processing unit, a field programmable gate array (FPGA), an application-specific integrated circuit (ASIC) or similar devices, which is not limited by the invention.

In the present embodiment, the output unit 160 may include the devices with functions of outputting information or outputting signals, such as a speaker or a display, which is not limited by the invention. The sensor 120 and the output unit 160 are connected or coupled to the processor 140 respectively.

It should be noted that, in another embodiment of the present invention, the electronic device 100 may further include a storage unit which is not shown in FIG. 2. The storage unit is coupled to the processor 140. In the present embodiment, the sensor 120, the processor 140 and the output unit 160 are disposed in the electronic device 100 to become a complete portable electronic device. However, in other embodiments, the sensor 120 may be a single isolated component that operated separately, and the processor 140 communicates with the sensor 120 via wired or wireless manner.

The sensor 120 or the electronic device 100 including the sensor 120 is adapted to worn by a user for evaluating a gait of the user. The user may wear the said sensor 120 or the said electronic device 100 on the chest, but the present invention is not limited thereto. In the present embodiment, the electronic device 100 may be a portable electronic device dedicated to perform the evaluation on the gait or physiological parameters. On the other hand, in other embodiments, the electronic device 100 may be implemented by a common smart portable device, but the present invention is not limited herein.

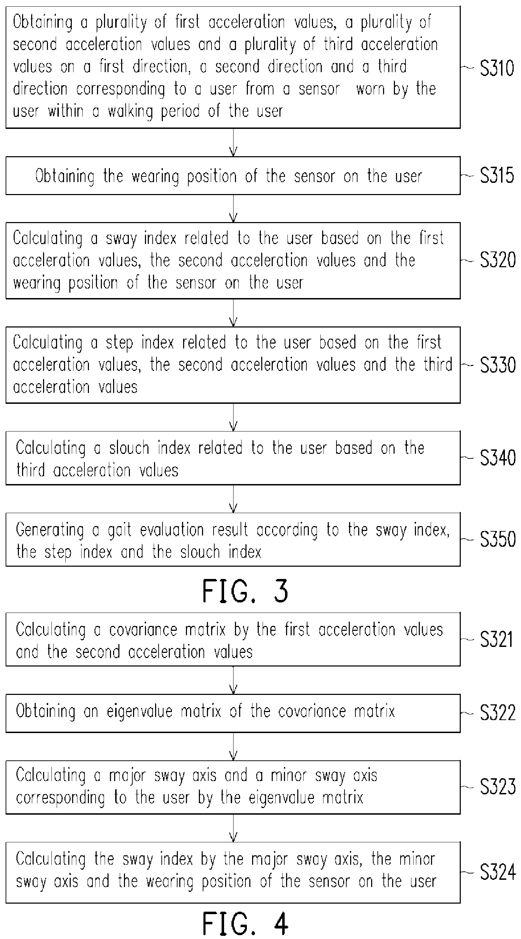

FIG. 3 is a flowchart of a gait evaluation method according to an embodiment of the invention. The gait evaluation method illustrated in FIG. 3 is adapted to the electronic device 100 illustrated in FIG. 2, but the object adaptable for the gait evaluation method is not limited herein. The sensor 120 or the electronic device 100 including the sensor 120 may be worn or equipped on the left chest or in the middle of the diaphragm of the user while performing the gait evaluation, but the present invention is not limited herein. The gait evaluation method illustrated in FIG. 3 is described as follows accompanying with the electronic device 100 illustrated in FIG. 2.

Referring to FIGs. 1, 2 and 3, at first, the sensor 120 detects a plurality of first acceleration values, a plurality of second acceleration values and a plurality of third acceleration values on a first direction, a second direction and a third direction corresponding to the user within a walking period of the user, and the processor 140 obtains the first acceleration values, the second acceleration values and the third acceleration values on the first direction, the second direction and the third direction corresponding to the user from the sensor 120 worn by the user (S310). The said walking period is, for example, 15 seconds or 20 seconds, but which is not limited herein. The sensor 120 continuously detects the changes of accelerations on the first direction, the second direction and the third direction corresponding to the user so as to obtain the first acceleration values, the second acceleration values and the third acceleration values. On the other hand, the processor 140 obtains a wearing position of the sensor 120 on the user (S315). To be specific, the processor 140 may determine the wearing position of the sensor 120 on the user according to strength of a connection between the processor 140 and the sensor 120 or according to a numerical type of the first, second and third acceleration values, but the present invention is not limited herein. In other embodiments of the present invention, the processor 140 may use a default position as the wearing position of the sensor 120 based on a setting. The default position is a body part on which the user wears the sensor 120 based on his/her habit or decision.

It should be noted that, the sensor 120 may read acceleration values on the first, second and third directions even if the user is standing still, due to the gravity or other factors. Therefore, the electronic device 100 may further correct to make sure that the detected first acceleration values, the detected second acceleration values and the detected third acceleration values can reflect the walking features and the gait of the user correctly.

In an embodiment of the present invention, the processor 140 further obtains a plurality of first reference values, a plurality of second reference values and a plurality of third reference values on the first direction, the second direction and the third direction corresponding to the user while the user wearing or equipping the sensor 120 or the electronic device 100 including the sensor 120 and before the user starting to walk. In other words, the first reference values, the second reference values and the third reference values are acceleration values on the first direction, the second direction and the third direction detected by the sensor 120 within a static period in which the user is static. The static period is, for example, 5 seconds, but which is not limited herein. Then, the processor 140 corrects the first acceleration values, the second acceleration values, and the third acceleration values detected within the said walking period by the sensor 120 according to the first reference values, the second reference values and the third reference values respectively. In the present embodiment, the processor 140 may perform correction base on an average of the first reference values, an average of the second reference values and an average of the third reference values, but the present invention is not limited herein.

In another embodiment of the present invention, the processor 140 corrects the first acceleration values, the second acceleration values, and the third acceleration values detected within the said walking period by the sensor 140 by using a first default reference, a second default reference and a third default reference directly, instead of measuring the first, second and third reference values previously.

It should be noted that, the first acceleration values, the second acceleration values, the third acceleration values, the first reference values, the second reference values, the third reference values, the first default reference, the second default reference and the third default reference may be all stored in the said storage unit, but the present invention is not limited herein.

Referring to FIGs. 1, 2 and 3 again, the processor 140 starts to calculate each of indexes after obtaining the first acceleration values, the second acceleration values and the third acceleration values from the sensor 120. Specifically, the processor 140 calculates a sway index related to the user based on the first acceleration values, the second acceleration values and the wearing position of the sensor 120 on the user (S320). On the other hand, the processor 140 calculates a step index related to the user based on the first acceleration values, the second acceleration values and the third acceleration values (S330). The processor 140 further calculates a slouch index related to the user based on the third acceleration values (S340). It should be noted that, although the sway index, the step index and the slouch index are sequentially calculated by the processor 140 in the present embodiment, the present invention is not limited herein. In other embodiments, the order of calculating the sway index, the step index and the slouch index may be changed correspondingly, or the processor 140 may calculate these indexes simultaneously.

FIG. 4 is a flowchart of calculating a sway index according to an embodiment of the invention. Specifically, the sway index represents a sway level of the user's body, and particularly represents the sway level on the first direction and the second direction shown in FIG. 1. Referring to FIG. 4, the processor 140 calculates a covariance matrix by the first acceleration values and the second acceleration values (S321). The covariance matrix $V$ is as follows.

$$V = \begin{bmatrix} C(Ax, Ax) & C(Ax, Ay) \\ C(Ay, Ax) & C(Ay, Ay) \end{bmatrix} \dots\dots\dots\dots (1)$$

$Ax$ is the first acceleration value, and $Ay$ is the second acceleration value. A plurality of first acceleration values $Ax$ and a plurality of second acceleration values $Ay$ are arranged according to a sampling time and used to calculate the covariance matrix $V$. After obtaining the covariance matrix $V$, the processor 140 further obtains an eigenvalue matrix $Ve$ of the covariance matrix $V$ (S322). The eigenvalue matrix $Ve$ is as follows.

$$Ve = \begin{bmatrix} Ve_0 \\ Ve_1 \end{bmatrix} \dots\dots\dots\dots (2)$$

After obtaining the eigenvalue matrix $Ve$, the processor 140 may calculate a major sway axis and a minor sway axis of a swaying trajectory of the user. In detail, it can be found that the user's body sways ovally on a virtual plane constituted by the first direction and the second direction according to an analysis on the first acceleration values $Ax$ and the second acceleration values $Ay$. Accordingly, the processor 140 further calculates the major sway axis $Do$ and the minor sway axis $D_1$ corresponding to the user by the eigenvalue matrix $Ve$ (S323). The major sway axis Do and the minor sway axis $D_1$ are as follows.

$$D_0 = (Ve_0)^2 \times 2.447 \dots\dots\dots\dots (3)$$

$$D_1 = (Ve_1)^2 \times 2.447 \dots\dots\dots\dots (4)$$

At last, the processor 140 calculates the sway index S by the major sway axis $Do$, the minor sway axis $D_1$ and the wearing position of the sensor 120 on the user (S324). In detail, it is enough to reflect the sway level of the user by the major sway axis Do and the minor sway axis $D_1$. However, there is a difference between the position of the sensor 120 or the electronic device 100 including the sensor 120 worn or equipped on the user, to an actual plane on which the user sways. In other words, the major sway axis $Do$ and the minor sway axis $D_1$ obtained from the first acceleration values $Ax$ and the second acceleration values $Ay$ detected by the sensor 120 are not on the same plane as the actual plane which the user sways on.

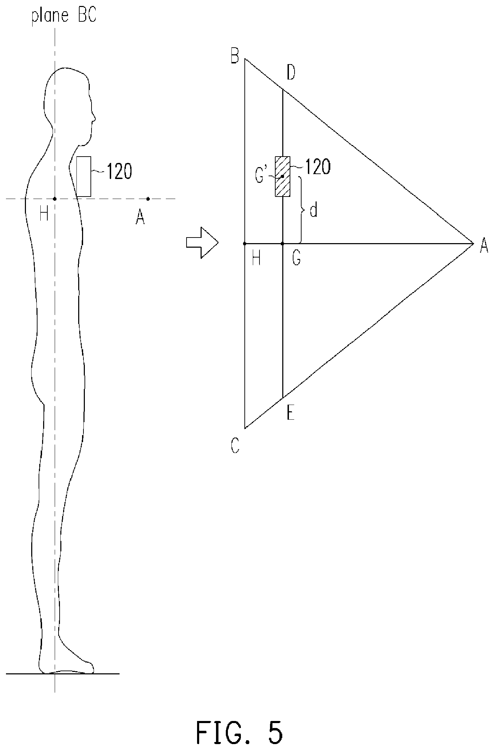

FIG. 5 is a schematic diagram of a regulation of a sway index according to an embodiment of the present invention. Referring to FIG. 5. The actual plane which the user sways on (plane BC) should be a median longitudinal plane of the user's body, but the sensor 120 is, for example, disposed on a position of the point G' due to the limitation of the user's body. In other words, the point G' is the wearing position of the sensor 120 on the plane DE of FIG. 5. In the present embodiment, the point G' is on the user's chest, for example. Besides, there is a slouch point H on the plane which the user sways on (plane BC). In general, the slouch point H is likely to shift if the user is slouched. A slouch median (line AH) extends from a reference point A to the slouch point H. The orthogonal projection of the point G' on the slouch median (line AH) is the point G, and there is an offset $d$ between the points G and G'. In addition, the distance between the reference point A and the slouch point H may be used as a correction ratio $a$ for correcting the major sway axis $D_0$ and the minor sway axis $D_1$. It can be found in FIG. 5 that the triangle ADE and the triangle ABC are similar to each other, and the correction ratio $a$ can be obtained from a proportional relation of the distance from the reference point A to the point G, the plane BC and the plan DE.

Due to the sensor 120 is not on the plane which the user's body sways on, errors may exist in the calculated major sway axis $D_0$ and the calculated minor sway axis $D_1$. Therefore, correction is required being performed. In the present embodiment, the processor 140 calculates the offset $d$ and the correction ratio $a$ according to the wearing position of the sensor on the user and the slouch median. It should be noted that, in other embodiments, the offset $d$ and the correction ratio $a$ may be just reference values set in the electronic device 100 by default. Then, the processor 140 obtains an initial sway index by calculating a square root of a sum of square of the major sway axis $Do$ and the minor sway axis $D_1$, and obtains a sway index S by regulating the initial sway index according to the offset $d$ and the correction ratio $a$. The sway index S is as follows.

$$S = \frac{\sqrt{(D_0{}^2 + D_1{}^2)} - d}{a} \ldots\ldots\ldots\ldots (5)$$

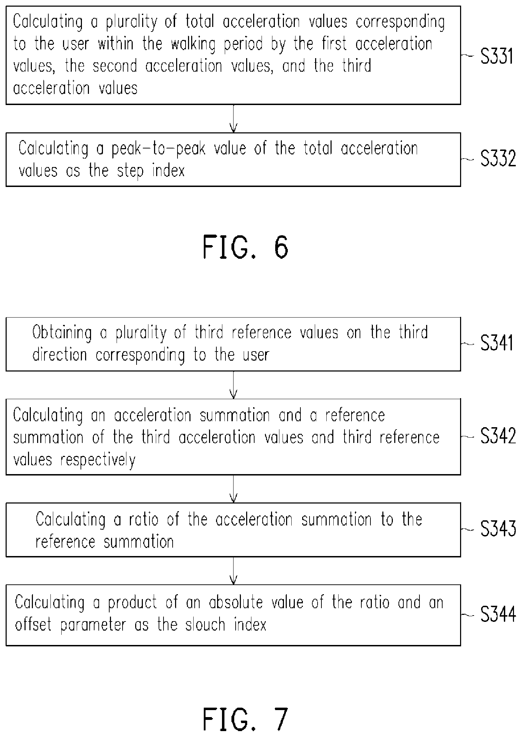

FIG. 6 is a flowchart of calculating a step index according to an embodiment of the invention. Specifically, the step index represents the step size of the user. Referring to FIG. 6, the processor 140 calculates a plurality of total acceleration values corresponding to the user within the walking period by the first acceleration values $Ax$, the second acceleration values $Ay$, and the third acceleration values $Az$ (S331). The total acceleration value $At$ is as follows.

$$At = \sqrt{Ax^2 + Ay^2 + Az^2} \ldots\ldots\ldots\ldots (6)$$

In detail, the total acceleration value $At$ may correspondingly be calculated by the first acceleration value $Ax$, the second acceleration value $Ay$ and the third acceleration value $Az$ obtained at the same sampling time. After obtaining a plurality of the total acceleration values $At$, the processor 140 further calculates a peak-to-peak value $Ap\text{-}p$ of the total acceleration values $At$ as the step index (S332).

Since the motion of human stepping is regular and periodic, the changes of the total acceleration values $At$ are also regular and periodic. Accordingly, in the present embodiment, the processor 140 determines whether the step size of the user is appropriate by the peak-to-peak value $Ap\text{-}p$ of the total acceleration values $At$.

FIG. 7 is a flowchart of calculating a slouch index according to an embodiment of the invention. Specifically, the slouch index represents the hump level of the user. Referring to FIG. 7, the processor 140 obtains a plurality of third reference values on the third direction corresponding to the user (S341). In detail, in the present embodiment, the third reference values may be a plurality of acceleration values measured on the third direction using the sensor 120 by the electronic device 100 within a static period before walking. After that, the processor 140 calculates an acceleration summation and a reference summation of the third acceleration values $Az$ and third reference values respectively (S342), calculates a ratio of the acceleration summation to the reference summation (S343), and calculates a product of an absolute value of the ratio and an offset parameter as the slouch index (S344). The slouch index $Slouch$ is as follows.

$$Slouch = \left| \frac{\sum_0^{S1} Az}{\sum_0^{S2} Bz} \right| \times 0.0207 \ldots\ldots\ldots\ldots (7)$$

The offset parameter represents an offset level of the third acceleration values measured by the electronic device 100 resulting from the hunchback of the user. In the present embodiment, the offset parameter is 0.0207, which is not limited in the present invention. $S1$ and $S2$ are respective lengths of sampling period of the third acceleration values $Az$ and the third reference values $Bz$. The processor 140 obtains a plurality of third acceleration values $Az$ and a plurality of third reference values $Bz$ according to the lengths of sampling period $S1$ and S2 respectively, so as to calculate the acceleration summation and the reference summation. In an embodiment of the present invention, the length of sampling period $S1$ may be a time length of the said walking period, and the length of sampling period $S2$ may be a time length of the said static period, but the present invention is not limited herein. In other embodiments, the lengths of sampling period $S1$ and $S2$ may be determined according to calculation requirements, but the length of sampling period $S1$ is usually longer than the length of sampling period $S2$. In another embodiment of the present invention, the reference summation may be further substituted by a default summation reference without requirement on measuring the third reference values before the user walks.

In detail, if the user becomes slouched while walking, the third acceleration value $Az$ on the third direction would be changed correspondingly. Therefore, the slouch index $Slouch$ may be used to evaluate a slouch level of the user by comparing the acceleration summation and the reference summation of the third acceleration values $Az$ and third reference values $Bz$.

Referring to FIGs. 1, 2 and 3 again, after obtaining the sway index $S$, the step index $Ap$ and the slouch index $Slouch$, the processor 140 generates a gait evaluation result according to the sway index $S$, the step index $Ap$ and the slouch index $Slouch$ (S350). Specifically, the processor 140 obtains a sway score, a step score and a slouch score by comparing the sway index $S$, the step index $Ap$ and the slouch index $Slouch$ with a plurality of thresholds respectively, and then generates the gait evaluation result by assigning respective weights to the sway score, the step score and the slouch score.

In the present embodiment, the processor 140 compares the sway index S with a first threshold. The sway level of the user is more serious when the sway index S exceeds the first threshold. The processor 140 sets the sway score as 0 in this case. Otherwise, the processor 140 sets the sway score as 1 when the sway index does not exceed the first threshold.

In the present embodiment, the processor 140 compares the step index *Ap* with a second threshold and a third threshold. In general, the step size of the user is moderate when the step index *Ap* is between the second threshold and the third threshold. The processor 140 sets the step score as 1 in this case. Otherwise, the processor 140 sets the step score as 0 when the step index *Ap* is smaller than the second threshold or larger than the third threshold. The second threshold and the third threshold are, for example, 0.281 and 0.506, which is not limited herein.

In the present embodiment, the processor 140 compares the slouch index *Slouch* with a fourth threshold. The slouch level of the user is more serious when the slouch index *Slouch* exceeds the fourth threshold. The processor 140 sets the slouch score as 0 in this case. Otherwise, the processor 140 sets the slouch score as 1 when the slouch index *Slouch* is smaller than or equal to the fourth threshold.

After obtaining the sway score, the step score and the slouch score, the processor 140 generates the gait evaluation result by respectively assigning different weights to the sway score, the step score and the slouch score based on the importance of the sway level, the step size and the slouch level. To be specific, in the present embodiment, for example, the weight of the sway score is 2, the weight of the step score is 1, and the weight of the slouch score is 4. A sum of the weighted sway score, the weighted step score and the weighted slouch score is regarded as the gait evaluation result. In general, the larger the numerical number of the gait evaluation result is, the more standard the gait of the user is.

In an embodiment of the present invention, the processor 140 further controls the output unit 160 outputting prompting information according to the gait evaluation result. Specifically, the prompting information may be the numerical number of the gait evaluation result, which is not limited by the invention. The processor 140 may further provide a compliment or a warning accordingly to a level of the gait evaluation result.

In summary, in the gait evaluation method and the related electronic device provided in the embodiments of the invention, a plurality of first acceleration values, a plurality of second acceleration values, and a plurality of third acceleration values detected within a walking period of the user and a wearing position of the sensor on a user are used to calculate a plurality of indexes of different types respectively, and a gait evaluation result is generated according to the indexes. By such, deviations of the sensor resulting from a wearing position thereof are corrected, and a gait of the user is evaluated accurately.

**Claims**

1. A gait evaluation method using a sensor (120) worn by a user for evaluating a gait of the user, comprising:

   obtaining a plurality of first acceleration values, a plurality of second acceleration values and a plurality of third acceleration values on a first direction, a second direction and a third direction corresponding to the user within a walking period of the user from the sensor (120) worn by the user (S310);
   obtaining a wearing position (G') of the sensor (120) on the user (S315);
   calculating a sway index related to the user based on the first acceleration values, the second acceleration values and the wearing position (G') of the sensor (120) on the user (S320);
   calculating a step index related to the user based on the first acceleration values, the second acceleration values and the third acceleration values (S330);
   calculating a slouch index related to the user based on the third acceleration values (S340); and
   generating a gait evaluation result according to the sway index, the step index and the slouch index (S350).

2. The gait evaluation method as claimed in claim 1, comprising:

   obtaining a plurality of first reference values, a plurality of second reference values and a plurality of third reference values on the first direction, the second direction and the third direction corresponding to the user before the user walks,
   and the step (S310) of obtaining the first acceleration values, the second acceleration values, and the third acceleration values further comprising:

      respectively correcting the obtained first acceleration values, the obtained second acceleration values, and the obtained third acceleration values according to the first reference values, the second reference values and the third reference values.

**3.** The gait evaluation method as claimed in claim 1, wherein the step (S320) of calculating the sway index further comprising:

calculating a covariance matrix by the first acceleration values and the second acceleration values (S321);
obtaining an eigenvalue matrix of the covariance matrix (S322);
calculating a major sway axis and a minor sway axis corresponding to the user by the eigenvalue matrix (S323); and
calculating the sway index by the major sway axis, the minor sway axis and the wearing position (G') of the sensor (120) on the user (S324).

**4.** The gait evaluation method as claimed in claim 3, wherein the step (S324) of calculating the sway index by the major sway axis and the minor sway axis further comprising:

calculating an offset ($d$) and a correction ratio according to the wearing position (G') of the sensor (120) on the user and a slouch median;
obtaining an initial sway index by calculating a square root of a sum of square of the major sway axis and the minor sway axis; and
obtaining the sway index by correcting the initial sway index according to the offset ($d$) and the correction ratio.

**5.** The gait evaluation method as claimed in claim 1, wherein the step (S330) of calculating the step index further comprising:

calculating a plurality of total acceleration values corresponding to the user within the walking period by the first acceleration values, the second acceleration values, and the third acceleration values (S331); and
calculating a peak-to-peak value of the total acceleration values as the step index (S332).

**6.** The gait evaluation method as claimed in claim 1, wherein the step (S340) of calculating the slouch index further comprising:

obtaining a plurality of third reference values on the third direction corresponding to the user (S341);
respectively obtaining an acceleration summation of the third acceleration values and a reference summation of the third reference values (S342);
calculating a ratio of the acceleration summation to the reference summation (S343); and
calculating a product of an absolute value of the ratio and an offset parameter as the slouch index (S344).

**7.** The gait evaluation method as claimed in claim 1, wherein the step (S350) of generating the gait evaluation result according to the sway index, the step index and the slouch index further comprising:

obtaining a sway score, a step score and a slouch score by comparing the sway index, the step index and the slouch index with a plurality of thresholds respectively; and
generating the gait evaluation result by assigning respective weights to the sway score, the step score and the slouch score.

**8.** The gait evaluation method as claimed in claim 1, further comprising:

outputting a prompting information according to the gait evaluation result.

**9.** An electronic device (100) adapted to evaluate a gait of a user, comprising:

a sensor (120), worn by the user, detecting a plurality of first acceleration values, a plurality of second acceleration values and a plurality of third acceleration values on a first direction, a second direction and a third direction corresponding to the user within a walking period of the user; and
a processor (140), connected to the sensor (120), the processor (140) obtains the first acceleration values, the second acceleration values and the third acceleration values on the first direction, the second direction and the third direction corresponding to the user from the sensor (120) worn by the user, and obtains a wearing position (G') of the sensor (120) on the user, the processor (140) calculates a sway index related to the user based on the first acceleration values, the second acceleration values and the wearing position (G') of the sensor (120) on the user; the processor (140) calculates a step index related to the user based on the first acceleration values, the second acceleration values and the third acceleration values; the processor (140) calculates a slouch index related to the user based on the third acceleration values; and the processor (140) generates a gait evaluation result according to the sway index, the step index and the slouch index.

10. The electronic device (100) as claimed in claim 9, wherein the processor (140) obtains a plurality of first reference values, a plurality of second reference values and a plurality of third reference values on the first direction, the second direction and the third direction corresponding to the user before the user walks,
and the processor (140) corrects the first acceleration values, the second acceleration values, and the third acceleration values detected by the sensor (120) according to the first reference values, the second reference values and the third reference values respectively.

11. The electronic device (100) as claimed in claim 9, wherein the processor (140) calculates a covariance matrix by the first acceleration values and the second acceleration values; the processor (140) obtains an eigenvalue matrix of the covariance matrix; the processor (140) calculates a major sway axis and a minor sway axis corresponding to the user by the eigenvalue matrix; and the processor (140) calculates the sway index by the major sway axis, the minor sway axis and the wearing position (G') of the sensor (120) on the user.

12. The electronic device (100) as claimed in claim 11, wherein the processor (140) calculates an offset ($d$) and a correction ratio according to the wearing position (G') of the sensor (120) on the user and a slouch median; the processor (140) obtains an initial sway index by calculating a square root of a sum of square of the major sway axis and the minor sway axis; and the processor (140) obtains the sway index by correcting the initial sway index according to the offset ($d$) and the correction ratio.

13. The electronic device (100) as claimed in claim 9, wherein the processor (140) calculates a plurality of total acceleration values corresponding to the user within the walking period by the first acceleration values, the second acceleration values, and the third acceleration values; and the processor (140) calculates a peak-to-peak value of the total acceleration values as the step index.

14. The electronic device (100) as claimed in claim 9, wherein the processor (140) obtains a plurality of third reference values on the third direction corresponding to the user; the processor (140) obtains an acceleration summation of the third acceleration values and a reference summation of the third reference values respectively; calculates a ratio of the acceleration summation to the reference summation; and the processor (140) calculates a product of an absolute value of the ratio and an offset parameter as the slouch index.

15. The electronic device (100) as claimed in claim 9, wherein the processor (140) obtains a sway score, a step score and a slouch score by comparing the sway index, the step index and the slouch index with a plurality of thresholds respectively; and the processor (140) generates the gait evaluation result by assigning respective weights to the sway score, the step score and the slouch score.

16. The electronic device (100) as claimed in claim 9, further comprising:

an output unit (160), coupled to the processor (140), wherein the processor (140) controls the output unit (160) outputting a prompting information according to the gait evaluation result.

sway level

slouch level

first direction

third direction

second direction

step size

FIG. 1

120 ~ sensor

140 ~ processor

output unit ~ 160

100

FIG. 2

Obtaining a plurality of first acceleration values, a plurality of second acceleration values and a plurality of third acceleration values on a first direction, a second direction and a third direction corresponding to a user from a sensor worn by the user within a walking period of the user ~ S310

Obtaining the wearing position of the sensor on the user ~ S315

Calculating a sway index related to the user based on the first acceleration values, the second acceleration values and the wearing position of the sensor on the user ~ S320

Calculating a step index related to the user based on the first acceleration values, the second acceleration values and the third acceleration values ~ S330

Calculating a slouch index related to the user based on the third acceleration values ~ S340

Generating a gait evaluation result according to the sway index, the step index and the slouch index ~ S350

# FIG. 3

Calculating a covariance matrix by the first acceleration values and the second acceleration values ~ S321

Obtaining an eigenvalue matrix of the covariance matrix ~ S322

Calculating a major sway axis and a minor sway axis corresponding to the user by the eigenvalue matrix ~ S323

Calculating the sway index by the major sway axis, the minor sway axis and the wearing position of the sensor on the user ~ S324

# FIG. 4

# FIG. 5

Calculating a plurality of total acceleration values corresponding to the user within the walking period by the first acceleration values, the second acceleration values, and the third acceleration values ~ S331

Calculating a peak-to-peak value of the total acceleration values as the step index ~ S332

# FIG. 6

Obtaining a plurality of third reference values on the third direction corresponding to the user ~ S341

Calculating an acceleration summation and a reference summation of the third acceleration values and third reference values respectively ~ S342

Calculating a ratio of the acceleration summation to the reference summation ~ S343

Calculating a product of an absolute value of the ratio and an offset parameter as the slouch index ~ S344

# FIG. 7

Europäisches
Patentamt

European
Patent Office

Office européen
des brevets

# EUROPEAN SEARCH REPORT

Application Number

EP 16 19 7015

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/123669 A1 (KINOSHITA SHIGEO [CN] ET AL) 16 May 2013 (2013-05-16) * abstract; figures 2, 7A-7B, 10A-10B, 12A-12B * * paragraphs [0062], [0074], [0135], [0173] * * the whole document * ----- | 1-16 | INV. A61B5/11 G06K9/00 A61B5/00 |
| A | US 2013/041291 A1 (SOUBEYRAT CYRILLE [FR] ET AL) 14 February 2013 (2013-02-14) * abstract; figure 4 * * paragraphs [0110] - [0113] * * the whole document * ----- | 1-16 | |

TECHNICAL FIELDS SEARCHED (IPC)

A61B
G06K

The present search report has been drawn up for all claims

| Place of search | Date of completion of the search | Examiner |
|---|---|---|
| The Hague | 10 May 2017 | Furlan, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 238 619 A1**

## ANNEX TO THE EUROPEAN SEARCH REPORT
## ON EUROPEAN PATENT APPLICATION NO.

EP 16 19 7015

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2017

| Patent document cited in search report | | | Publication date | Patent family member(s) | | | Publication date |
|---|---|---|---|---|---|---|---|
| US 2013123669 | A1 | | 16-05-2013 | CN | 103025241 | A | 03-04-2013 |
| | | | | DE | 112011102491 | T5 | 29-05-2013 |
| | | | | JP | 5724237 | B2 | 27-05-2015 |
| | | | | JP | 2012024449 | A | 09-02-2012 |
| | | | | US | 2013123669 | A1 | 16-05-2013 |
| | | | | WO | 2012014714 | A1 | 02-02-2012 |
| US 2013041291 | A1 | | 14-02-2013 | EP | 2560551 | A1 | 27-02-2013 |
| | | | | FR | 2959112 | A1 | 28-10-2011 |
| | | | | US | 2013041291 | A1 | 14-02-2013 |
| | | | | WO | 2011131497 | A1 | 27-10-2011 |

For more details about this annex : see Official Journal of the European Patent Office, No. 12/82